# EUROPÄISCHE PATENTSCHRIFT

(11) **EP 2 704 632 B1**
(45) Veröffentlichungstag und Bekanntmachung des Hinweises auf die Patenterteilung: **29.05.2019**
(21) Anmeldenummer: 12705282.7
(22) Anmeldetag: 17.02.2012
(51) Int. Cl.: A61B 5/00, A61B 5/1459, A61B 5/1455

(54) **MESSVORRICHTUNG ZUR MESSUNG ZEREBRALER PARAMETER**
MEASURING DEVICE FOR MEASURING CEREBRAL PARAMETERS
DISPOSITIF DE MESURE POUR LA MESURE DE PARAMÈTRES CÉRÉBRAUX

(30) Priorität: 05.05.2011 CH 774112011
(43) Veröffentlichungstag der Anmeldung: 12.03.2014
(73) Patentinhaber: Luciole Medical AG, 8005 Zürich (CH)
(72) Erfinder: FRÖHLICH, Jürg Hans, 8006 Zürich (CH); MUSER, Markus Hugo, 8820 Wädenswil (CH)
(74) Vertreter: BOVARD AG
(86) Internationale Anmeldenummer: PCT/EP2012/052778
(87) Internationale Veröffentlichungsnummer: WO 2012/150054

(56) Entgegenhaltungen:
- WO-A1-99/37204
- WO-A1-2005/082225
- WO-A2-2010/015094
- US-A- 4 730 622
- US-A1- 2007 019 916
- US-B1- 6 261 226
- US-B1- 6 315 712

## Beschreibung

Die Erfindung betrifft eine Messvorrichtung zur Messung von Parametern eines Körpergewebes mit einer länglichen Sonde zum Einführen in das Körpergewebe nach dem Oberbegriff des Patentanspruchs 1, insbesondere eine Messvorrichtung zur Messung zerebraler Parameter mittels eines invasiven Messverfahrens.

### STAND DER TECHNIK

Es sind verschiedene invasive Verfahren zur zerebralen Diagnostik und Therapie bekannt, bei welchen unterschiedliche zerebrale Parameter gemessen werden, wie etwa Parameter der zerebralen Hämodynamik. Es werden beispielsweise Parameter bzgl. der Konzentration von desoxygeniertem und oxygeniertem Hämoglobin, der mittleren Durchgangszeit eines Indikators, des zerebralen Blutvolumens, des zerebralen Blutflusses oder des Gewebesauerstoffindexes gemessen. Zu derartigen Verfahren gehören z. B. die Nahinfrarotspektroskopie-Messung (NIRS) oder die Pulsoxymetrie. Zur Erfassung zerebraler Parameter können z. B. subdurale Messungen, Messungen auf der Gehirnoberfläche, ventrikuläre Messungen oder intraparenchymale Messungen durchgeführt werden.

Aus der EP 1301119 B1 ist z. B. eine Subduralsonde und eine Vorrichtung zur Messung der zerebralen Hämodynamik und Sauerstoffsättigung bekannt. Hierfür umfasst die Sonde einen ersten optischen Leiter, der Licht zum distalen Ende der Sonde und somit zum Kopf und ins Gehirn eines Patienten leitet, und einen zweiten optischen Leiter, der Licht vom distalen Ende zu einer proximal gelegenen Verarbeitungseinrichtung führt. Die optischen Leiter sind im Wesentlichen parallel zueinander angeordnet. Am distalen Ende der optischen Leiter sind optische Einheiten vorgesehen, welche durch die optischen Leiter geführtes Licht aus der Transmissionsrichtung entlang der Leiter um einen Winkel von z. B. 90° ablenken. Das Licht wird dadurch z. B. durch die optischen Einheiten von einer Richtung parallel zur Dura vertikal in das Gehirngewebe umgeleitet. Die optischen Leiter und die optischen Einheiten bilden emittierende und empfangende Optoden und sind von einer Umhüllung mit länglicher Form umgeben. Der Abstand der optischen Einheiten bestimmt die Implantationstiefe, bis zu welcher Licht in das Gewebe eindringen und zurückgestreut werden kann. Durch das Hirngewebe reflektiertes oder gestreutes Licht wird auf die zweite optische Einheit gerichtet und dadurch aus einer Richtung annähernd senkrecht zu der Transmissionsrichtung in den optischen Leiter eingespeist und in Transmissionsrichtung zur Verarbeitungseinrichtung geleitet. Das Licht tritt bei einer solchen Sonde in radialer Richtung seitlich aus und reflektiertes und gestreutes Licht wird auch in radialer Richtung auf der gleichen Seite wieder eingefangen. Dabei kann ein signifikanter Teil des Lichts unmittelbar an der Seite der Sonde entlang deren Oberfläche zur empfangenden Optode gelangen ohne durch das zu vermessende Gewebe zu dringen. Dadurch kann das Messsignal gestört und verfälscht werden. Ferner ist bei einer Messung seitlich der Sonde die Lokalisierung des vermessenen Gewebebereichs schwierig.

Aus der US 5,579,774 ist ferner eine Vorrichtung zur Messung des Blutflusses in zerebralen Bereichen bekannt, die senkrecht in ein zerebrales Gewebe eingeführt werden kann. Die Vorrichtung weist entlang einer länglichen Messsonde mehrere Sensoren zur Durchführung einer Laser-Doppler-Flowmetrie Messung auf. Die Sensoren sind an Öffnungen entlang der Länge und des Umfangs der Messsonde und an deren Spitze vorgesehen. Die Sensoren werden mittels optischer Leiter an eine Lichtquelle angeschlossen, die am Ende eine reflektierende Oberfläche aufweisen, die das Licht im Wesentlichen senkrecht zur Messsonde in das umliegende Gewebe lenkt. Der Abstand der Sensoren muss dabei derart gross sein, dass Licht, das von einem Sensor emittiert und vom Gewebe reflektiert oder gestreut wird, nicht von einem anderen Sensor erfasst werden kann. Das emittierte und das empfangene Licht werden daher bei dieser Messvorrichtung an der gleichen Öffnung in der Messsonde ausgesendet und eingefangen. Dadurch wird der messbare Bereich innerhalb des zerebralen Gewebes stark eingeschränkt und die Messungen können durch das Zusammenliegen von Sende- und Empfangsöffnung verfälscht werden.

Die US 4,986,671 zeigt eine optische Messsonde zur Messung von Druck, Temperatur und Flussgeschwindigkeit in Blutgefässen. Die Messsonde kann an einem Katheter vorgesehen werden, der in die Blutgefässe eingeführt wird. Die Messsonde weist einen optischen Leiter auf, der Licht unterschiedlicher Wellenlänge zur Messung der verschiedenen Parameter zur Spitze der Sonde leitet. An der Spitze der Sonde ist ein elastisches optisches Element vorgesehen, das eine nach innen konvexe Oberfläche bildet, die mit unterschiedlichen Materialien beschichtet ist. Licht das durch den optischen Leiter zur elastischen Oberfläche des optischen Elements geführt wird, wird dort reflektiert. Die zu messenden Parameter können aus dem Licht ermittelt werden, das an der deformierten elastischen Oberfläche reflektiert wurde, wobei die Deformation charakteristisch ist für die zu messenden Parameterwerte. Bei diesem Messverfahren tritt kein Licht aus der Messsonde in das umliegende Gewebe aus, so dass der Messbereich der Sonde im umliegenden Bereich sehr klein ist. Ferner können einige relevante Parameter, wie etwa der Sauerstoffgehalt, mit einer solchen Messsonde nicht ermittelt werden.

In der US 6,261,226 B1 wird ein elektronisch steuerbares Endoskop beschrieben, das an seinem distalen Ende mehrere Messfelder aufweist. Jedes Messfeld umfasst ein Beleuchtungsmittel und ein zugehöriges Detektionsmittel, die über Lichtleiter an eine Verarbeitungseinheit angeschlossen sind. Es sind diverse Messfelder entlang des Umfangs und an der Spitze des Endoskops vorgesehen, so dass die Austrittsbereich der Beleuchtungsmittel seitlich am Endoskop und am distalen Ende vorgesehen sind. Die Detektionsmittel, die den Beleuchtungsmitteln zugeordnet sind, sind unmittelbar neben dem Beleuchtungsmittel angeordnet und im Wesentlichen in gleicher Richtung orientiert, wodurch das Messsignal negativ beeinflusst werden kann.

In der WO 2010/015094 wird ein Katheter zur Messung der Durchblutung in einem Körpergewebe beschrieben, wobei an einem Umfangsbereich eines Mittelstücks ein optischer Leiter als Lichtemitter zur Emission eines Lichtstrahls vorgesehen ist, welcher an einer der Lichtaustrittsfläche gegenüberliegenden Reflexionsfläche in das Körpergewebe umgelenkt und der an dem Körpergewebe absorbierte und reflektierte Lichtstrahl über die Reflexionsfläche reflektiert wieder in den optischen Leiter eingekoppelt wird. Aufgrund der unmittelbaren Nähe von Austritts- und Empfangsfläche ist das Messvolumen und die Menge an reflektiertem und empfangenem Licht gering, so dass auch die Sensitivität gering ist.

### AUFGABE DER ERFINDUNG

Es ist eine Aufgabe der vorliegenden Erfindung eine Messvorrichtung zur invasiven Messung von Parametern eines Körpergewebes zu schaffen, die eine zuverlässige und möglichst genaue Messung von unterschiedlichen Parametern eines Körpergewebes ermöglicht,
eine Lokalisierung des vermessenen Bereichs vereinfacht, bei der Messung das umliegende Gewebe nur minimal beeinträchtigt und einen einfachen Aufbau aufweist.

Diese und weitere Aufgaben werden von einer Messvorrichtung zur Messung von Parametern eines Körpergewebes nach dem unabhängigen Patentanspruch 1 gelöst. Besondere Ausgestaltungen und/oder Varianten gehen aus den Unteransprüchen hervor.

Eine Messvorrichtung zur Messung von Parametern eines Körpergewebes nach der vorliegenden Erfindung weist eine längliche Sonde zum Einführen in das Körpergewebe auf. Die Sonde kann z. B. eine zylindrische oder auch eine abgeflachte Form aufweisen. Die Sonde weist einen steifen Abschnitt am distalen Ende auf, wie etwa eine steife Umhüllung oder ein steifes Gehäuse, mit dem sie in das Körpergewebe eingeführt werden kann. Als steif ist ein solcher Abschnitt bereits dann zu verstehen, wenn eine ausreichende Stabilität vorliegt, um die Sonde in das Körpergewebe einführen zu können. In der Regel ist ein solcher Abschnitt steifer als ein sich anschliessendes Elemente der Sonde, wie etwa ein Katheterschlauch oder dergleichen, kann aber dennoch eine gewisse Flexibiltät oder Nachgiebigkeit aufweisen, um ein sanftes Einführen zu ermöglichen. Der steife Abschnitt kann an einem Ende einer flexiblen Leitung, wie etwa an einem Katheterschlauch, angebracht sein. Es ist wenigstens ein optischer oder elektrischer Leiter zur Übermittlung von Lichtstrahlen vorgesehen, der im Wesentlichen entlang der Längsachse der Sonde innerhalb der Sonde verläuft und z. B. durch die flexible Leitung zur Sonde geführt wird. Die Sonde umfasst einen Austrittsbereich, aus dem aus dem optischen Leiter emittierte Lichtstrahlen aus der Sonde in das Körpergewebe austreten, wonach die Lichtstrahlen am Körpergewebe reflektiert, gestreut und/oder absorbiert werden. Weiter weist die Sonde wenigstens einen Empfangsbereich auf, durch den im Körpergewebe reflektierte und/oder gestreute Eingangslichtstrahlen z. B. auf einen Photodetektor und/oder in einen optischen Leiter in der Sonde eintreten. Für den Fall des optischen Leiters sind getrennte optische Leiter für einen emittierten Lichtstrahl und einen reflektierten, bzw. gestreuten Eingangslichtstrahl innerhalb der Sonde vorgesehen. Es ist jedoch nicht ausgeschlossen, dass ein optischer Leiter einen Lichtstrahl, der zur Messung der Parameter aus der Sonde austritt, und ebenso einen Lichtstrahl, der im Körpergewebe reflektiert oder gestreut wurde, übermitteln kann. Der wenigstens eine optische Leiter oder im Falle des Photodetektors mindestens eine elektrische Leiter kann die Eingangslichtstrahlen als Messsignal zu einer Verarbeitungseinrichtung übermitteln, die aus den Eingangslichtstrahlen die zu messenden Parameter bestimmt. Dies kann z. B. in bekannter Weise durch Analyse der Lichtintensität oder der Wellenlängen der Eingangsstrahlen im Vergleich zu den emittierten Lichtstrahlen erfolgen. Die Verarbeitungsvorrichtung kann auch zur Steuerung der emittierten Lichtstrahlen verwendet werden.

Nach der vorliegenden Erfindung ist der Austrittsbereich am distalen Ende der Sonde vorgesehen und in Längsrichtung der Sonde orientiert. Er ist somit in Strahlrichtung der in dem optischen Leiter geführten Lichtstrahlen orientiert. Der Austrittsbereich kann an einer Frontseite der Sonde liegen. Der Austrittsbereich kann zumindest annähernd symmetrisch um die Längsachse ausgebildet sein und den distalen Abschluss der Sonde bilden. Vorzugsweise fällt die Flächennormale des Austrittsbereichs mit der Längsachse der Sonde zusammen. Die aus dem Austrittsbereich emittierten Lichtstrahlen treten in Längsrichtung der Längsrichtung der Sonde aus dieser in distaler Richtung aus. Demnach weist die Ausbreitungsrichtung der emittierten Lichtstrahlen zumindest einen Vektoranteil auf, der parallel zur Längsachse der Sonde, bzw. des emittierenden optischen Leiters, in Richtung des vor der Sonde liegenden Körpergewebes ausgerichtet ist. Die emittierten Lichtstrahlen können ein Bündel von Lichtstrahlen bilden, die beispielsweise geradlinig entlang der Längsachse aus dem Austrittsbereich austreten oder auch symmetrisch um die Längsachse, z. B. unter einem Winkel zur Längsachse kegelartig ausgestrahlt werden.

Der wenigstens eine Empfangsbereich ist beabstandet zum distalen Ende der Sonde und somit auch beabstandet zum Austrittsbereich an einem seitlichen Umfangsbereich der Sonde vorgesehen. Somit ist der Empfangsbereich in radialer Richtung zur Längsachse ausgebildet. Der Empfangsbereich kann z. B. durch eine Öffnung oder ein transparentes Fenster im Mantel der Sonde gegeben sein. Der Empfangsbereich dient dazu, am Körpergewebe gestreutes oder reflektiertes Licht einzufangen. Die Eingangsstrahlen treffen hierfür beispielsweise auf einen Photodetektor, der am Empfangsbereich vorgesehen und an einen elektrischen Leiter angeschlossen ist, oder werden mittels eines optischen Elements, das in der Sonde angeordnet ist, in einen optischen Leiter eingespeist. Es können mehrere Empfangsbereiche rings um die Sonde oder in Längsrichtung der Sonde verteilt vorgesehen sein. Dabei kann für jeden
Empfangsbereich ein eigener elektrischer oder optischer Leiter vorgesehen sein oder es wird ein gemeinsamer optischer Leiter verwendet. Mit dem Empfangsbereich können vorzugsweise sowohl Eingangslichtstrahlen aufgenommen werden, die im Wesentlichen radial zur Sondenachse, also senkrecht zum Empfangsbereich, verlaufen als auch annähernd parallel zur Sondenachse verlaufen sowie alle in einem Winkel zwischen diesen Ausrichtungen verlaufenden Eingangsstrahlen.

Bei einem Verfahren zur Messung von Parametern eines Körpergewebes mit einer oben beschriebenen Messvorrichtung treten aus der Sonde, bzw. aus dem Lichtleiter emittierte Lichtstrahlen zumindest teilweise in Längsrichtung am distalen Ende der Sonde aus dieser aus und treten beabstandet zu diesem Austrittsbereich an einem seitlichen Umfangsbereich der Sonde wieder in diese ein, wobei die an einem Empfangsbereich eintretenden Eingangslichtstrahlen relativ zu aus dem Austrittsbereich emittierten Lichtstrahlen einer Umlenkung von mehr als 90° unterliegen. Da die Ausbreitungsrichtung der emittierten Lichtstrahlen zumindest einen kleinen Vektoranteil aufweisen, der von der Sonde distal nach vorne ausgerichtet ist, also weg vom wenigstens einen Empfangsbereich, müssen sie um mehr als 90° umgelenkt werden, um einen Empfangsbereich zu erreichen und als Messsignal aufgenommen zu werden. Der überwiegende Teil der emittierten Lichtstrahlen wird in der Regel entlang der Längsachse der Sonde emittiert, so dass überwiegend eine Umlenkung um mehr als 190° notwendig ist, damit die Eingangslichtstrahlen auf den Empfangsbereich treffen können. Es wird daher sichergestellt, dass die emittierten Lichtstrahlen eine Umlenkung durch Reflexion oder Streuung am Körpergewebe erfahren haben, bevor sie als Eingangslichtstrahlen am Empfangsbereich eintreffen. Ein Austritt aus der Sonde und unmittelbarer Eintritt in die Sonde, ohne dass eine Interaktion mit dem Körpergewebe statt gefunden hat, wie es bei herkömmlichen Messsonden z. B. dadurch erfolgen kann, dass der Empfangsbereich und der Austrittsbereich identisch sind oder dass die emittierten Lichtstrahlen entlang der Sonde etwa innerhalb eines Führungskanals für die Sonde verlaufen ohne in das Körpergewebe einzutreten, kann ausgeschlossen werden. Die Lichtstrahlen können keinen Bypass-Verlauf nehmen.

In der Regel tritt ein überwiegender Teile der emittierten Lichtstrahlen zumindest annähernd entlang der Längsachse der Sonde, bzw. dem optischen Leiter, aus der Sonde aus und vorzugsweise annähernd radial zur Längsachse der Sonde am Empfangsbereich wieder in diese ein. Somit unterliegt ein überwiegender Teil der emittierten Lichtstrahlen bis zum Eintritt an einem Empfangsbereich einer Umlenkung in einem Bereich um 270°.

Bei einer Messvorrichtung nach der vorliegenden Erfindung können daher der Austrittsbereich und der Eingangsbereich nahe beieinander liegen und die Sonde kann kürzer gebaut werden, ohne dass dadurch das Messsignal nachteilig beeinflusst wird. Das Körpergewebe wird somit beim Einführen der Messvorrichtung weniger beansprucht. Durch eine symmetrische Anordnung des Austrittsbereichs und der Eingangsbereiche um die Längsachse der Sonde kann der Bereich des Gewebes, der vermessen wird, in einfacher Weise durch Lokalisierung der Position der Sonde bestimmt werden. Es ist nicht erforderlich eine Ausrichtung der Sonde an einer bestimmten Position zu erfassen. Mit dem Verfahren können beispielsweise Parameter in Bezug auf den Bluttfluss, Hämoglobinwerte, das im Hirngewebe eingelagerte Wasser oder diverse Marker erfasst werden.

Sofern die Ausrichtung der Sonde bekannt ist und einzelne elektrische oder optische Leiter für verschiedene Empfangsbereiche verwendet werden, kann die Auswertung von Parametern für bestimmte Teilbereiche um die Sonde erfolgen, die einem Empfangsbereich zugeordnet sind. Die Auswertung der Parameter kann damit verfeinert werden.

Bei einer Ausführungsform der Messvorrichtung ist ein Abstand zwischen einem Austrittsbereich und einem Empfangsbereich zwischen 1 und 40 mm, bevorzugt zwischen 3 und 20 mm, vorgesehen. Vorzugsweise sind mehrere Empfangsbereiche in gleichem Abstand zum Austrittsbereich um den Umfang der Sonde verteilt angeordnet. Es können beispielsweise zwei sich diametral gegenüberliegende Empfangsbereiche oder drei, vier oder mehr vorzugsweise symmetrisch um den Umfang verteilt angeordnete Empfangsbereiche vorgesehen sein. Der feste Abschnitt der Sonde, z. B. in Form einer steifen Umhüllung oder eines festen Gehäuses, weist in einer Ausführungsform maximal eine Länge von 50mm, vorzugsweise weist er eine Länge im Bereich von 20 mm und besonders bevorzugt von 12 mm auf.

In einer Variante kann der Austrittsbereich z. B. als Austrittsöffnung oder Austrittsfenster im Sondengehäuse oder einer Sondenumhüllung vorgesehen sein, durch welche die aus dem Lichtleiter emittierten Lichtstrahlen aus der Sonde austreten.

In einer anderen Variante der Messvorrichtung kann der Austrittsbereich ein optisches Element umfassen, welches das distale Ende der Sonde abschliesst. Das optische Element kann eine konvexe oder kegelartig nach aussen gerichtete Oberfläche aufweisen, wobei sich der radiale Durchmesser in distaler Richtung verjüngt. Aus dem optischen Leiter emittierte Lichtstrahlen verlaufen somit durch das optische Element bevor sie aus der Sonde austreten. Das optische Element kann selbst den Austrittsbereich bilden. Das optische Element kann aus demselben Material, wie eine Umhüllung um die Sonde oder wie das Sondengehäuse, bestehen. Beispielsweise kann das optische Element aus Epoxidharz oder einem in Bezug auf seine optischen Eigenschaften vergleichbaren Material, wie z. B. Polycarbonat, Glas, Acrylat, Polypropylen, Polyimid bestehen. Es ist vorteilhaft, wenn das optische Element aus einem Material mit einem höheren Brechungsindex als das angrenzende Gewebe oder die angrenzende Materie besteht. Somit treten aus dem optischen Leiter austretende Lichtstrahlen, die durch das optische Element verlaufen, an der Oberfläche des optischen Elements derart aus, dass sie von der Längsachse der Sonde weg gebeugt werden. Grundsätzlich ist auch die Verwendung eines Gitters als optisches Element denkbar, das beispielsweise in ein Material eingelassen ist, welches über dem optischen Leiter angeordnet wird. Vorzugsweise wird der emittierte Lichtstrahl an dem optischen Element am distalen Ende der Sonde zu einem Lichtkegel aufgeweitet, der einen grösseren Bereich des Körpergewebes erfasst, als ein direkt aus einem optischen Leiter austretender Lichtstrahl. Das optische Element lenkt die aus dem optischen Leiter emittierten Lichtstrahlen somit in Richtung des wenigstens einen Empfangsbereichs derart ab, dass ein Vektoranteil der Ausbreitungsrichtung in distaler Richtung verbleibt. Das optische Element ist vorzugsweise symmetrisch um die Längsachse der Sonde ausgebildet und bildet den distalen Abschluss der Sonde, der in das Körpergewebe vordringt. Der am weitesten distal gelegene Bereich des optischen Elements ist vorzugsweise stumpf, bzw. gewölbt ausgeformt, z. B. kugelförmig oder elliptisch. Das optische Element kann aus Vollmaterial bestehen. Es kann die Austrittsöffnung des emittierenden optischen Leiters versiegeln. Grundsätzlich kann das optische Element eine Beschichtung auf seiner Oberfläche aufweisen, welche beispielsweise das Einführen der Sonde in das Körpergewebe erleichtert oder die Beugung der Lichtstrahlen unterstützt.

Ferner kann die Messvorrichtung an einem distalen Endbereich der Sonde eine Reflexionsfläche mit einer Öffnung aufweisen, durch die der optische Leiter mündet. Die emittierten Lichtstrahlen werden dann durch die Öffnung geführt und in distaler Richtung hinter der Reflexionsfläche emittiert werden. Die Reflexionsfläche kann z. B. einen Hohlraum der Sonde, der den wenigstens einen optischen Leiter aufnimmt, abschliessen. Ein Ende des Lichtstrahlen emittierenden optischen Leiters kann ein kleines Stück durch die Reflexionsfläche hindurch ragen oder bündig mit dieser abschliessen. Die Reflexionsfläche ist vorzugsweise senkrecht zur Längsachse der Sonde, bzw. zur Strahlrichtung des optischen Leiters, ausgerichtet. Die Reflexionsfläche dient dazu Lichtstrahlen, die in Richtung des Austrittsbereichs reflektiert oder gestreut werden, in Richtung des Körpergewebes zurück zu reflektieren.

Zur Messung der Parameter im Körpergewebe können Lichtstrahlen mit unterschiedlichen Wellenlängen verwendet werden. Vorzugsweise wird Licht im Infrarot-Bereich mit einer oder mehreren Wellenlängen im Bereich zwischen 600 nm und 1000 nm verwendet. Es können auch Lichtstrahlen mit verschiedenen bestimmten Wellenlängen verwendet werden, die zeitlich versetzt und gepulst in das Körpergewebe abgesendet werden.

Grundsätzlich können auch weitere Sensoren an der Sonde vorgesehen sein, wie beispielsweise Sensoren zur Temperatur- oder Druckmessung.

Eine Messvorrichtung nach der vorliegenden Erfindung ist besonders zur Messung von zerebralem Gewebe, geeignet, indem die Sonde zumindest annähernd senkrecht zur Schädeloberfläche in das Gewebe eingeführt wird. Da die Abstrahlung der Lichtstrahlen zumindest teilweise in distaler Richtung, d.h. in Einführrichtung der Sonde in das Gewebe, erfolgt, muss die Sonde weniger weit in das Gewebe eingebracht werden, als bei einer radialen Abstrahlung, um den gleichen Gewebebereich vermessen zu können. Eine Schädigung des Gewebes durch das Einführen der Sonde wird dadurch verringert.

### KURZE BESCHREIBUNG DER ZEICHNUNGEN

Bevorzugte Ausführungsformen der Erfindung werden im Folgenden anhand der Zeichnungen dargestellt, die lediglich zur Erläuterung dienen und nicht einschränkend auszulegen sind. Aus den Zeichnungen offenbar werdende Merkmale der Erfindung sollen einzeln und in jeder Kombination als zur Offenbarung der Erfindung gehörend betrachtet werden. In den Zeichnungen zeigen:
Fig. 1: drei-dimensionale Darstellung einer Messvorrichtung nach der vorliegenden Erfindung,
Fig. 2: Längsschnitt durch eine Messvorrichtung nach Figur 1, und
Fig. 3: Detailansicht eines distalen Endes einer Messvorrichtung mit einem optischen Element.

Im Folgenden wird unter einer distalen Richtung diejenige Richtung verstanden, in die ein Ende einer Messvorrichtung weist, das in ein Körpergewebe eingeführt wird. Dieses Ende ist somit das distale Ende.

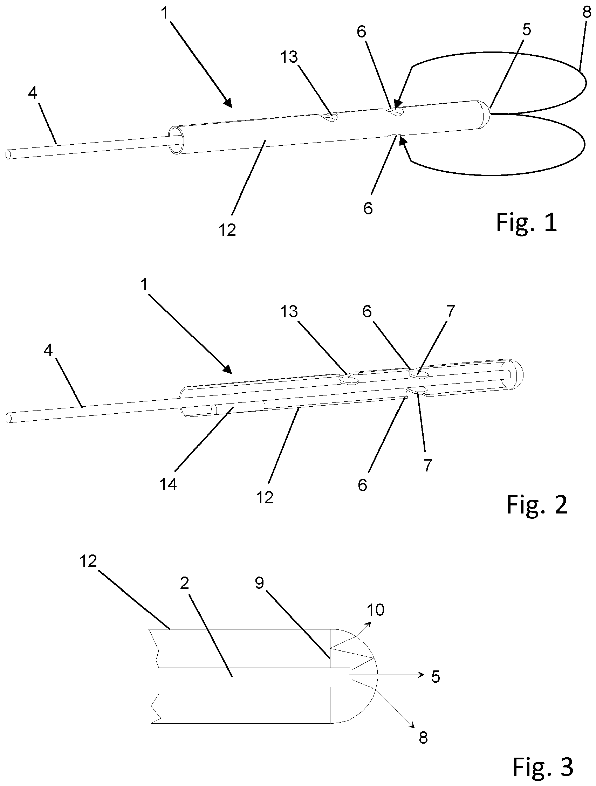

In den Figuren 1 und 2 ist eine Messvorrichtung zur Messung von Parametern eines Körpergewebes mit einer länglichen Sonde 1 zum Einführen in das Körpergewebe gezeigt. Die Messvorrichtung umfasst wenigstens einen ersten optischen Leiter 2 und einen zweiten optischen oder einen elektrischen Leiter 3, die in einer Leitung 4 durch ein festes zylindrisches Gehäuse 12 der Sonde 1 geführt werden. Der erste optische Leiter 2 übermittelt Lichtstrahlen von einer Lichtquelle (nicht gezeigt) an ein distales Ende der Sonde 1. Die Sonde 1 weist einen Austrittsbereich 5, aus welchem aus dem optischen Leiter emittierte Lichtstrahlen des ersten optischen Leiters aus der Sonde in das Körpergewebe austreten. Weiter weist die Sonde 1 zwei gegenüber liegende Empfangsbereiche 6 auf, durch welche im Körpergewebe reflektierte und/oder gestreute Lichtstrahlen als Eingangslichtstrahlen in die Sonde 1 eintreten. Die Eingangslichtstrahlen werden durch den zweiten optischen oder einen elektrischen Leiter 3 übermittelt. Es ist beispielsweise eine Verarbeitungseinrichtung (nicht gezeigt), etwa in Form eines Computers, vorgesehen, der die Emission von Lichtstrahlen steuert und die Eingangslichtstrahlen als Messsignal empfängt und diese derart verarbeitet, dass daraus Parameter des Körpergewebes ermittelt werden. Ferner umfasst die Messsonde einen Drucksensor 13 und einen Temperatursensor 14.

Der Austrittsbereich 5 am distalen Ende der Sonde 1 ist in Längsrichtung der Sonde orientiert, d. h. eine Flächennormale oder eine Symmetrieachse des Austrittsbereichs 5 weist in Richtung der Längsachse. Emittierte Lichtstrahlen treten somit zumindest teilweise in Längsrichtung der Sonde aus dieser aus. Die gegenüber liegenden Empfangsbereiche 6 sind an einem seitlichen Umfangsbereich der Sonde 1 vorgesehen und von dem Austrittsbereich 5 beabstandet angeordnet. Die Empfangsbereiche 6 weisen z. B. eine Photozelle 7 auf, mit der die Eingangslichtstrahlen erfasst werden können. Die Empfangsbereiche 6, bzw. die Photozellen, sind in der dargestellten Ausführungsform in radialer Richtung zur Längsachse der Sonde orientiert, d. h. ihr Flächennormale steht senkrecht zur Längsachse der Sonde. Der Abstand zwischen dem Austrittsbereich 5 und den Empfangsbereichen 6 beträgt beispielsweise 5 mm.

Wie in Figur 1 ersichtlich ist, tritt ein Bündel 8 von emittierten Lichtstrahlen zu einem grossen Teil im Wesentlichen in Längsrichtung der Sonde 1 aus und in das in distaler Richtung vor der Sonde 1 liegende Körpergewebe ein. Am Körpergewebe werden die Lichtstrahlen gestreut, reflektiert und/oder absorbiert und somit aus ihrer ursprünglichen Richtung abgelenkt und in ihrer Intensität verändert. Ein Teil der abgelenkten Lichtstrahlen tritt an den Empfangsbereichen 6 wieder in die Sonde 1 ein und wird als Messsignal an die Verarbeitungseinrichtung übermittelt. Da die emittierten Lichtstrahlen zumindest teilweise in distaler Richtung ausgestrahlt werden, müssen sie mindestens eine Umlenkung von 90° erfahren, um zu einem der Empfangsbereichen 6 gelangen zu können.

In Figur 3 ist eine weitere Ausführungsform einer Messvorrichtung nach der vorliegenden Erfindung gezeigt, bei der am distalen Ende der Sonde 1 eine Reflexionsfläche 9 und ein darüber angeordnetes optisches Element 10 vorgesehen ist. Die Reflexionsfläche 9 schliesst einen inneren Hohlraum 11 der Sonde 1 in distaler Richtung ab, wobei der erste optische Leiter 2 ein kleines Stück durch eine Öffnung der Reflexionsfläche 9 hindurch geführt ist und vor dem optischen Element 10 endet. Die Reflexionsfläche 9 weist zumindest auf der distalen Seite eine reflektierende Oberfläche auf, die geeignet ist, die von der Lichtquelle erzeugten Lichtstrahlen zu reflektieren. Die emittierten Lichtstrahlen werden durch das Material des optischen Elements 10 gestrahlt und zumindest beim Austritt aus der Oberfläche des optischen Elements 10 von der Längsachse der Sonde weg gebeugt. Hierfür ist das optische Element 10 aus einem Material vorgesehen, das optisch dichter ist als die angrenzende Materie des Körpergewebes und es weist eine nach aussen konvexe Oberfläche auf, die symmetrisch um die Längsachse verläuft. Das optische Element 10 lenkt somit die emittierten Lichtstrahlen um, bevor sie aus dem Austrittsbereich der Sonde austreten. Sofern ein Teil der Lichtstrahlen an der Oberfläche des optischen Elements zurück reflektiert werden, können diese an der Reflexionsfläche 9 reflektiert und somit wieder in das Körpergewebe zurück gestrahlt werden.

### BEZUGSZEICHENLISTE

1 Sonde
2 erster optischer Leiter
3 zweiter optischer Leiter
4 Leitung
5 Austrittsbereich
6 Empfangsbereich
7 Photozelle
8 Strahlbündel
9 Reflexionsfläche
10 optisches Element
11 Hohlraum
12 Gehäuse
13 Drucksensor
14 Temperatursensor

## Patentansprüche

1. Messvorrichtung zur Messung von Parametern eines Körpergewebes mit einer länglichen Sonde (1) zum Einführen in das Körpergewebe, umfassend:
- wenigstens einen optischen Leiter (2) zur Übermittlung von Lichtstrahlen, der entlang der Längsachse der Sonde innerhalb der Sonde verläuft,
- einen Austrittsbereich (5), aus welchem aus dem optischen Leiter emittierte Lichtstrahlen aus der Sonde in das Körpergewebe austreten, wobei die emittierten Lichtstrahlen eine oder mehrere Wellenlängen im Bereich zwischen 600 nm und 1000 nm aufweisen, und
- wenigstens einen Empfangsbereich (6), durch welchen im Körpergewebe reflektierte und/oder gestreute Lichtstrahlen, die aus dem Austrittsbereich (5) ausgetreten sind als Eingangslichtstrahlen auf einen Photodetektor oder in einen optischen Leiter (2;3) in der Sonde eintreten, **dadurch gekennzeichnet, dass**
- der Austrittsbereich (5) am distalen Ende der Sonde (1) vorgesehen und in Längsrichtung der Sonde (1) orientiert ist, so dass emittierte Lichtstrahlen in Längsrichtung der Längsachse der Sonde aus dieser austreten, und
- der wenigstens eine Empfangsbereich (6) beabstandet zum Austrittsbereich (5) an einem seitlichen Umfangsbereich radial zur Längsachse der Sonde vorgesehen ist.

2. Messvorrichtung nach Anspruch 1, **dadurch gekennzeichnet, dass** mehrere Empfangsbereiche (6) in Umfangsrichtung um die Sonde (1) verteilt vorgesehen sind.

3. Messvorrichtung nach einem der vorhergehenden Ansprüche, **dadurch gekennzeichnet, dass** ein Bündel (8) von emittierten Lichtstrahlen im Wesentlichen derart fokussiert ist, dass diese symmetrisch um die Längsachse der Sonde (1) aus dem optischen Leiter (2) austreten.

4. Messvorrichtung nach einem der vorhergehenden Ansprüche, **dadurch gekennzeichnet, dass** ein Abstand zwischen einem Austrittsbereich (5) und einem Empfangsbereich (6) zwischen 1 und 40 mm beträgt.

5. Messvorrichtung nach einem der vorhergehenden Ansprüche, **dadurch gekennzeichnet, dass** die Sonde (1) einen steifen Abschnitt (12) aufweist, der maximal eine Länge von 50 mm aufweist.

6. Messvorrichtung nach einem der vorhergehenden Ansprüche, **dadurch gekennzeichnet, dass** der Austrittsbereich (5) ein optisches Element (10) umfasst, welches das distale Ende der Sonde (1) abschliesst.

7. Messvorrichtung nach Anspruch 6, **dadurch gekennzeichnet, dass** das optische Element (10) aus einem Material mit einem grösseren Brechungsindex als eine angrenzende Materie des Körpergewebes besteht.

8. Messvorrichtung nach einem der Ansprüche 6 oder 7, **dadurch gekennzeichnet, dass** das optische Element (10) aus Epoxidharz und/oder z. B. Polycarbonat, Glas, Acrylat, Polypropylen, Polyimid besteht.

9. Messvorrichtung nach einem der vorhergehenden Ansprüche, **dadurch gekennzeichnet, dass** eine Reflexionsfläche (9) mit einer Öffnung vorgesehen ist, durch die der optische Leiter (2) mündet, wobei die emittierten Lichtstrahlen durch die Öffnung geführt und in distaler Richtung hinter der Reflexionsfläche emittiert werden.

10. Messvorrichtung nach Anspruch 9, **dadurch gekennzeichnet, dass** das optische Element (10) in distaler Richtung hinter der Reflexionsfläche (9) angeordnet ist.

## Claims

1. Measuring device for measuring parameters of a body tissue using an elongated probe (1) for insertion into the body tissue, comprising:
- at least one optical guide (2) for transmitting light rays that runs along the longitudinal axis of the probe within the probe,
- an exit region (5), from which light rays emitted from the optical guide exit out of the probe, into the body tissue, whereby the emitted light rays have one or more wavelengths in the range between 600 nm and 1000 nm, and
- at least one receiving region (6), through which light rays reflected and/or scattered in the body tissue, which have exited out of the exit region (5), enter as input light rays on a photo detector or in an optical guide (2;3) in the probe, **characterized in that**
- the exit region (5) is provided at the distal end of the probe (1), and is oriented in the longitudinal direction of the probe (1), so that emitted light rays exit the probe in longitudinal direction of the longitudinal axis thereof, and
- the at least one receiving region (6) is provided, at a distance from the exit region (5), on a lateral circumferential region radial to the longitudinal axis of the probe.

2. The measuring device according to claim 1, **characterized in that** a multiplicity of receiving regions (6) is provided distributed around the probe (1) in circumferential direction.

3. The measuring device according to one of the preceding claims, **characterized in that** a bundle (8) of emitted light rays is focused substantially in such a way that these light rays exit out of the optical guide (2) symmetrically around the longitudinal axis of the probe (1).

4. The measuring device according to one of the preceding claims, **characterized in that** a spacing between an exit region (5) and a receiving region (6) measures between 1 and 40 mm.

5. The measuring device according to one of the preceding claims, **characterized in that** the probe (1) has a rigid section (12) having maximally a length of 50 mm.

6. The measuring device according to one of the preceding claims, **characterized in that** the exit region (5) comprises an optical element (10) which terminates the distal end of the probe (1).

7. The measuring device according to claim 6, **characterized in that** the optical element (10) is made of a material having a greater refraction index than an adjacent material of the body tissue.

8. The measuring device according to one of the claims 6 or 7, **characterized in that** the optical element (10) is made of epoxy resin and/or e.g. polycarbonate, glass, acrylate, polypropylene, polyimide.

9. The measuring device according to one of the preceding claims, **characterized in that** a reflection surface (9) is provided with an opening through which the optical guide (2) comes out, the emitted light rays being emitted guided through the opening and in distal direction behind the reflection surface.

10. The measuring device according to claim 9, **characterized in that** the optical element (10) is disposed in distal direction behind the reflection surface (9).

## Revendications

1. Dispositif de mesure pour la mesure de paramètres d'un tissu corporel comportant une sonde allongée (1) destinée à être insérée dans le tissu corporel, comprenant :
- au moins un guide optique (2) pour transmettre des faisceaux lumineux qui courent au sein de la sonde le long de l'axe longitudinal de celle-ci,
- une zone de sortie (5), depuis laquelle les faisceaux lumineux émis par le guide optique sortent de la sonde et pénètrent dans le tissu corporel, les faisceaux lumineux émis possédant une ou plusieurs longueurs d'onde comprises entre 600 nm et 1000 nm, et
- au moins une zone de réception (6), via laquelle les faisceaux lumineux réfléchis et/ou dispersés dans le tissu corporel, qui sont sortis de la zone de sortie (5), pénètrent en tant que faisceaux lumineux d'entrée sur un photo-détecteur ou dans un guide optique (2 ; 3) dans la sonde,
**caractérisé en ce que**
- la zone de sortie (5) est prévue au niveau de l'extrémité distale de la sonde (1), et est orientée dans la direction longitudinale de la sonde (1), de telle sorte que les faisceaux lumineux émis sortent de la sonde dans la direction longitudinale selon l'axe longitudinal de cette dernière, et
- au moins une des zones de réception (6) est prévue dans une zone latérale circonférentielle décalée par rapport à la zone de sortie (5) et agencée radialement par rapport à l'axe longitudinal de la sonde.

2. Dispositif de mesure selon la revendication 1, **caractérisé en ce que** plusieurs zones de réception (6) sont réparties sur la circonférence de la sonde (1).

3. Dispositif de mesure selon l'une des revendications précédentes, **caractérisé en ce qu'**un ensemble de faisceaux (8) parmi les faisceaux lumineux émis est focalisé substantiellement de telle sorte que ces derniers sortent du guide optique (2) symétriquement autour de l'axe longitudinal de la sonde (1).

4. Dispositif de mesure selon l'une des revendications précédentes, **caractérisé en ce que** l'espacement entre une zone de sortie (5) et une zone de réception (6) mesure entre 1 mm et 40 mm.

5. Dispositif de mesure selon l'une des revendications précédentes, **caractérisé en ce que** la sonde (1) possède une partie rigide (12) ayant une longueur d'au maximum 50 mm.

6. Dispositif de mesure selon l'une des revendications précédentes, **caractérisé en ce que** la zone de sortie (5) comprend un élément optique (10) qui clôture l'extrémité distale de la sonde (1).

7. Dispositif de mesure selon la revendication 6, **caractérisé en ce que** l'élément optique (10) est fait d'un matériau ayant un indice de réfraction plus grand qu'un matériau adjacent du tissu corporel.

8. Dispositif de mesure selon l'une des revendications 6 ou 7, **caractérisé en ce que** l'élément optique (10) est constitué d'une résine époxy et/ou par exemple de polycarbonate, de verre, d'acrylate, de polypropylène ou de polyimide.

9. Dispositif de mesure selon l'une des revendications précédentes, **caractérisé en ce qu'**une surface de réflexion (9) est munie d'une ouverture à travers laquelle le guide optique (2) débouche, les faisceaux lumineux émis étant émis par guidage à travers l'ouverture et selon une direction distale derrière la surface de réflexion.

10. Dispositif de mesure selon la revendication 9, **caractérisé en ce que** l'élément optique (10) est disposé selon une direction distale derrière la surface de réflexion (9).
